(19) **Europäisches Patentamt**
**European Patent Office**
**Office européen des brevets**

(11) **EP 3 117 766 B1**

(12) **EUROPEAN PATENT SPECIFICATION**

(45) Date of publication and mention
of the grant of the patent:
**24.02.2021 Bulletin 2021/08**

(51) Int Cl.:
*A61B 5/021* (2006.01)    *A61B 5/0295* (2006.01)
*A61B 5/024* (2006.01)    *A61B 5/08* (2006.01)

(21) Application number: **15177174.8**

(22) Date of filing: **16.07.2015**

(54) **PROCESSING BIOLOGICAL DATA**

VERARBEITUNG BIOLOGISCHER DATEN

TRAITEMENT DE DONNÉES BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.01.2017 Bulletin 2017/03**

(73) Proprietor: **Preventicus GmbH**
**07743 Jena (DE)**

(72) Inventor: **HÜBNER, Thomas**
**07749 Jena (DE)**

(74) Representative: **Fish & Richardson P.C.**
**Highlight Business Towers**
**Mies-van-der-Rohe-Straße 8**
**80807 München (DE)**

(56) References cited:
**WO-A1-2014/022906    WO-A1-2014/031082
US-A1- 2010 228 102    US-A1- 2013 079 606
US-A1- 2013 184 596    US-B2- 8 398 556**

- **Y. ZHAO ET AL: "Determining Blood Pressure
  Changes and Vascular Stiffness State Using
  Optical Pulse Pressure Analysis", BIOMEDICAL
  ENGINEERING / BIOMEDIZINISCHE TECHNIK, 7
  January 2013 (2013-01-07), XP055206023, ISSN:
  0013-5585, DOI: 10.1515/bmt-2013-4188**

- **GREEN GEOFFREY C ET AL: "Continuous
  multiorgan variability analysis to track severity
  of organ failure in critically ill patients",
  JOURNAL OF CRITICAL CARE, vol. 28, no. 5, 31
  October 2013 (2013-10-31), XP028711912, ISSN:
  0883-9441, DOI: 10.1016/J.JCRC.2013.04.001**
- **SCULLY C G ET AL: "Physiological Parameter
  Monitoring from Optical Recordings With a
  Mobile Phone", IEEE TRANSACTIONS ON
  BIOMEDICAL ENGINEERING, IEEE SERVICE
  CENTER, PISCATAWAY, NJ, USA, vol. 59, no. 2,
  1 February 2012 (2012-02-01), pages 303-306,
  XP011408628, ISSN: 0018-9294, DOI:
  10.1109/TBME.2011.2163157**
- **Y. ZHAO ET AL: "Applanation Tonometry for
  Determining Arterial Stiffness", BIOMEDICAL
  ENGINEERING / BIOMEDIZINISCHE TECHNIK,
  vol. 57, no. SI-1 Track-F, 30 January 2012
  (2012-01-30), XP055206071, ISSN: 0013-5585,
  DOI: 10.1515/bmt-2012-4121**
- **MOHAMED ELGENDI: "On the Analysis of
  Fingertip Photoplethysmogram Signals",
  CURRENT CARDIOLOGY REVIEWS, vol. 8, no. 1,
  1 June 2012 (2012-06-01), pages 14-25,
  XP055206723, ISSN: 1573-403X, DOI:
  10.2174/157340312801215782**
- **ANNA A. AHIMASTOS ET AL: "Gender
  Differences in Large Artery Stiffness Pre- and
  Post Puberty", JOURNAL OF CLINICAL
  ENDOCRINOLOGY & METABOLISM, vol. 88, no.
  11, 1 November 2003 (2003-11-01), pages
  5375-5380, XP055206763, ISSN: 0021-972X, DOI:
  10.1210/jc.2003-030722**

**Description**

**Technical Field**

The present invention relates to processing of biological data. Based on pulse waveform analysis, data pertaining to, for example, the heart rate, respiratory rate, and/or blood pressure of a human subject are determined and processed.

**Background Art**

The primary causes for diseases such as heart attack and stroke are conditions that are often hard to detect and do not entail pronounced symptoms. For example, hypertension and coronary artery disease (CAD) are among the primary causes for heart attack and atrial fibrillation (AFIB) is one of the primary causes for stroke. Regular measurement of blood pressure, heart rate, respiratory rate and a detailed analysis of such biological parameters of a subject can be employed in detecting hypertension, AFIB, CAD, and other conditions or the early onset thereof. However, these measures are often not employed on a regular basis.

Blood pressure is the pressure exerted by circulating blood upon the walls of blood vessels and is one of the principal vital signs of a person. It is regulated by the nervous and endocrine systems and varies depending on a number of factors including current activity and general health condition of a person. Pathologically low blood pressure is referred to as hypotension, and pathologically high blood pressure is referred to as hypertension. Both pathologies can have different causes and can range from mild to severe, with both acute and chronic forms. Chronic hypertension is a risk factor for many complications, including peripheral vascular disease, heart attack, and stroke. Both hypertension and hypotension are often undetected for longer periods of time because of infrequent monitoring.

Hypertension is generally more common and constitutes the predominant risk factor for a cardiovascular disease and associated health problems including death, higher than those for smoking and diabetes. One major problem with hypertension is that high blood pressure does not necessarily entail pronounced symptoms and that, consequently, there are many people living their lives without realizing that they have elevated or high blood pressure. Measuring and monitoring blood pressure can be done in a number of ways, including at home, as an outpatient, or as an inpatient. However, sporadic and/or infrequent measurements are typically not meaningful enough for effective early detection of hypertension and associated diseases, due to the intervals between measurements often being too long and the measurements being done not often enough.

Medical professionals commonly measure arterial pressure using a sphygmomanometer, which historically used the height of a column of mercury to reflect the circulating pressure, and blood pressure values are typically reported in millimeters of mercury (mm Hg). For each heartbeat, blood pressure varies between systolic and diastolic pressures. Systolic pressure is the peak pressure in the arteries, occurring near the end of a cardiac cycle when the ventricles are contracting. Diastolic pressure is the minimum pressure in the arteries, occurring near the beginning of a cardiac cycle when the ventricles are filled with blood. Typical normal measured values for a resting and healthy adult are 120 mm Hg systolic pressure and 80 mm Hg diastolic pressure (i.e. 120/80 mm Hg).

Systolic and diastolic arterial blood pressures are not static but undergo natural variations from one heartbeat to the next and throughout the day (in a circadian rhythm). Variations occur in response to stress or exercise, changes in nutrition, and disease or associated medication. Blood pressure is one of the four main vital signs, further including body temperature, respiratory rate, and pulse rate, that are routinely monitored by medical professionals and healthcare providers.

Blood pressure can be measured in a noninvasive manner, including palpation, auscultatory or oscillometric methods, continuous noninvasive techniques (CNAP), and based on the pulse wave velocity (PWV) principle. Measuring blood pressure invasively, for example using intravascular cannulae, can produce very accurate measurements, but is much less common due to its invasive nature and is typically restricted to inpatient treatment.

Blood pressure in humans is significantly affected by the elasticity of the vascular system. The elasticity of the vascular system of a person depends on different factors including age, but also on the presence or absence of particular diseases or illnesses. If, for example, the elasticity of the vascular system of a patient decreases due to old age or due to the patient suffering from arteriosclerosis, the blood pressure of the patient increases.

The heart rate of a subject and the respiratory rate of a subject can also be determined by a physician using known methods for inpatient treatment. Also these measurements are typically taken only at irregular intervals and/or with long periods of time without measurements in between.

The variability of certain biological parameters, such as heart rate, respiration, blood pressure, can serve as an indicator for medical conditions, for example sleep apnea, depression, AFIB, CAD. It is noted that the term variability can mean a single variability value or measure or a plurality of values indicative of the variability of the respective parameter. Any known representations of variabilities are accepted within the scope of the present documents.

A. Seeck, W. Rademacher, C. Fischer, J. Haueisen, R. Surber, A. Voss, "Prediction of atrial fibrillation recurrence after cardioversion-Interaction analysis of cardiac autonomic regulation" have found in a study that the assessment of the autonomic regulation by analyzing the coupling of heart rate and systolic blood pressure provides a potential tool for the prediction of arterial fibrillation recurrence after CV and could aid in the adjustment of therapeutic options for patients with arterial fibrillation.

W. Poppe et al., "Eignen sich die Hüllungskurven von Arterienpulswellen für eine Fernbeurteilung psychotischer Krankheitsverläufe?", have found that the envelope of the arterial pulse wave can be indicative of a subject being classified with respect to a particular psychosis and further indicative of a likely progression of a psychosis. This research applies to, for example, the correlation of depression with pulse wave data. US 2010/228102 discloses systems and methods for monitoring a correlation between heart rate and blood pressure in a patient. When a characteristic of the correlation exceeds a threshold, a patient status indicator signal is sent to a monitoring device. The patient status indicator signal can indicate a particular medical condition or alerts a care provider to a change in status. The heart rate signal can be used to improve a blood pressure estimate generated by a different signal. In some embodiments, the heart rate, blood pressure and correlation signals are used in a predictive mathematical model to estimate patient status or outcome.

US 2013/079606 A1 discloses a patient monitoring system, which may receive a PPG signal including samples of a pulse waveform. The PPG signal demonstrates morphology changes based on respiration. The system calculates morphology metrics from the PPG signal, the first and/or second derivatives thereof. The morphology metrics demonstrate amplitude modulation, baseline modulation, and frequency modulation of the PPG signal that is related to respiration. Morphology metric signals generated from the morphology metrics may be used to determine respiration information such as respiration rate.

WO 2014/031082 discloses a method and device for quantifying heart rate variability coherence of a subject. The method comprises obtaining a bio-signal from a subject. Further, the method includes correlating a time-domain heart rate variability signal with a sine wave representing a time domain reference heart rate variability signal to obtain a correlated heart rate variability signal. This includes adjusting frequency of the sine wave and performing cross-correlation between the sine wave at each of the adjusted frequencies and the heart rate variability signal to obtain a correlated signal. The method includes quantifying the heart rate variability coherence based on the correlated variability signal.

US 8,398,556 discloses systems and methods for non-invasive continuous blood pressure determination. A PPG signal is received and locations of pulses within the PPG signal are identified. An area within a particular pulse is measured, e.g. the upstroke, downstroke or the entire pulse, and may be measured relative to a time-domain axis or a baseline of the pulse. The pulse may be split into multiple sections and the area of each section may be measured. The area of one portion of the pulse may correspond to systolic blood pressure while the area of another portion may correspond to diastolic blood pressure. Empirical data may be used to determine blood pressure from the measured area by applying calibration data.

An aim of the present invention is to provide an apparatus for accurately determining biological parameters of a subject, for example heart rate, respiration, blood pressure, and the variabilities thereof, in a noninvasive manner, easily, and efficiently. It is a further aim to provide an apparatus for determining the biological parameters of a subject and the variabilities thereof with an improved accuracy.

A further aim of the present invention is to provide an apparatus for performing the non-invasive method for determining the blood pressure of a human subject. In particular, the apparatus is a mobile device, and preferably a conventional smart phone provided with a light source and an optical sensor.

## Summary of invention

According to the invention, there is provided an apparatus for determining a medical condition of a human subject, the apparatus comprising a control unit; and a means for providing pulse wave data representative of a heart beat of the human subject; wherein the control unit is configured to perform the steps of: receiving the pulse wave data; selecting a portion of the pulse wave data indicative of a plurality of heart periods; for the portion of the pulse wave data indicative of a plurality of heart periods: - determining a blood pressure variability based on the pulse wave data of the portion of the pulse wave data indicative of a plurality of heart periods; - determining a respiratory rate variability based on the pulse wave data of the portion of the pulse wave data indicative of a plurality of heart periods; and - determining a heart rate variability based on the pulse wave data of the portion of the pulse wave data indicative of a plurality of heart periods; and determining at least one correlation value based on at least one of the blood pressure variability, the respiratory rate variability, the heart rate variability, and a respective reference value; and determining a medical condition of the subject based on the at least one correlation value.

In an aspect, the pulse wave data indicative of a plurality of heart periods relates to a plurality of heart periods in direct succession to one another.

In a further aspect, the step of determining the respiratory rate variability based on the pulse wave data of the portion of the pulse wave data indicative of a plurality of heart periods comprises: determining a plurality of maxima based on the pulse wave data, the plurality of maxima denoting the maximum amplitude of a respective plurality of heart periods; determining a respiratory signal indicative of the respiratory rate based on the plurality of maxima, optionally including determining the respiratory signal based on a spline interpolation of the plurality of maxima; and determining the respiratory rate variability based on a time difference between each maximum of the respiratory signal.

In a further aspect, the step of determining the heart rate variability based on the pulse wave data of the portion of the pulse wave data indicative of a plurality of heart periods comprises: determining a plurality of reference points based on the pulse wave data, the plurality of reference points corresponding to a respective component of the plurality of heart periods, optionally the respective component being one of a maximum amplitude of the heart period, a rising edge of the heart rate amplitude; determining the heart rate variability based on a time difference between each reference point of the plurality of reference points.

Further according to the invention, the subject has a body height, an age, and a gender, and the step of determining the blood pressure variability comprises determining a plurality of blood pressure values, the step of determining a plurality of blood pressure values comprising, for each respective blood pressure value of the plurality of blood pressure values, each respective blood pressure value being associated with a respective heart period of the plurality of heart periods: - determining a systolic component of the respective heart period; - approximating the systolic component with a first Gaussian function and a second Gaussian function; and - determining a time difference (WWT) between the first and second Gaussian functions; and determining a respective blood pressure value (BP) of the plurality of blood pressure values of the subject based on the time difference (WWT), the body height, and/or the age.

Further according to the invention,

- the step of determining a plurality of blood pressure values comprises, for each blood pressure value of the plurality of blood pressure values:

    determining a preliminary stiffness index ($SI_p$) based on the body height and the time difference (WWT); determining an adjusted stiffness index ($SI_a$) based on the preliminary stiffness index ($SI_p$) and the age; and determining the blood pressure value (BP) based on the adjusted stiffness index ($SI_a$) and a regression model.

In a further aspect,

- the portion of the pulse wave data is indicative of a plurality of heart periods, and wherein the step of determining the time difference (WWT) further comprises: determining the time difference (WWT) for the plurality of successive heart periods as an average value based on the respective time differences determined for the plurality of heart periods; optionally the average value being the median value of the determined respective time differences; and/or
- the first and second Gaussian functions have a respective maximum amplitude, the maximum amplitude of the first Gaussian function being greater than or equal to the maximum amplitude of the second Gaussian function; and/or
- the first and second Gaussian functions have respective first and second standard deviations ($\sigma_1$, $\sigma_2$), the first and second standard deviations ($\sigma_1$, $\sigma_2$) being equal to each other.

In a further aspect, the step of approximating the systolic component comprises fitting the first and second Gaussian functions to the systolic component using

$$F(a,b,c,d,f) = \sum_{i=1}^{N}\left(S_i - \left(a \cdot e^{-\frac{1}{2}\left(\frac{t-b}{c}\right)^2} + d \cdot e^{-\frac{1}{2}\left(\frac{t-f}{c}\right)^2}\right)\right)^2 \overset{!}{=} min$$

with $a$, $b$, $c$, and d being determined using non-linear optimization or curve-fitting.

In a further aspect, the regression model comprises a regression function $f(SI_a, g) = BP_{sys}$, where $SI_a$ is the adjusted stiffness index ($SI_a$), g is the gender of the subject, and $BP_{sys}$ is the blood pressure; and wherein determining the blood pressure value comprises determining the blood pressure value based on the regression function, optionally wherein the regression function comprises a linear function of the type $f(x) = ax + b$, wherein a ranges from 1 to 20 mmHg/(m/s) and $b$ ranges from 0 to 80 mmHg, more preferably wherein a ranges from 5 to 15 mmHg/(m/s) and $b$ ranges from 20 to 60 mmHg.

In a further aspect, determining the adjusted stiffness index ($SI_a$) is based on an adjustment function $f(SI_p) = SI_a$, where $SI_p$ is the preliminary stiffness index and $SI_a$ is the adjusted stiffness index ($SI_a$), optionally wherein the adjustment function is a linear function of the type $f(x) = cx + d$, where c and d are adjustment factors determined based on a plurality of value pairs comprising an age value and an associated stiffness index value; optionally wherein

$$c = \frac{SI - \mu}{range(age)}$$ with $\mu$ = 0,109 $*$ *age* + 3,699 and *range*(*age*) = 0,1663 $*$ *age* + 4,3858 - $\mu$, *age* being the age of the subject, and d = 0.

In a further aspect, determining the systolic component comprises: determining a respective global maximum of the respective heart period; determining the second order derivative of the respective heart period; determining a maximum value of the second order derivative located at least at a predetermined time difference from the global maximum; and defining the systolic component as a portion of the heart period between the start of the heart period and the maximum value; optionally the predetermined time difference to the global maximum being 350 ms or less, further optionally the predetermined time difference to the global maximum being 250 ms or less.

In a further aspect, determining the preliminary stiffness index (SI$_p$) is based on a function $SI_p = \frac{h}{WWT}$ , where $h$ is the subject height, *WWT* is the time difference, and $SI_p$ is the preliminary stiffness index (SI$_p$).

In a further aspect, the step of determining at least one correlation value is based on the heart rate variability; the step of determining at least one correlation value further comprising: generating, based on a plurality of heart rate variability values, a frequency distribution indicative of the distribution of the plurality of heart rate variability values in the time domain; determining a plurality of expected values; determining an entropy value indicative of a plurality of expected values, the entropy value being indicative of the medical condition of the subject.

In a further aspect, the frequency distribution indicative of the distribution of the plurality of heart rate variability values comprises a histogram, optionally wherein the histogram has a bin size of 8 ms.

In a further aspect, the portion of the pulse wave data indicative of a plurality of heart periods covers a period of between 2 minutes and 5 minutes; and the step of determining the variabilities of the blood pressure, respiratory rate, and the heart rate variability, is based on substantially all heart beats comprised in the pulse wave data.

In a further aspect, the step of determining at least one correlation value is based on the heart rate variability and the respiration rate variability, and wherein the step of determining at least one correlation value comprises detecting a correspondence between the heart rate variability and the respiration rate variability.

In a further aspect, the average value is the median value of the determined respective time differences.

In a further aspect, the first and second Gaussian functions have a respective maximum amplitude, the maximum amplitude of the first Gaussian function being greater than or equal to the maximum amplitude of the second Gaussian function.

In a further aspect, the first and second Gaussian functions have respective first and second standard deviations, the first and second standard deviations being equal to each other.

In a further aspect, determining the systolic component comprises determining a respective global maximum of the respective heart period; determining the second order derivative of the respective heart period; determining a maximum value of the second order derivative located at least at a predetermined time difference from the global maximum; and defining the systolic component as a portion of the heart period between the start of the heart period and the maximum value.

In a further aspect, the predetermined time difference to the global maximum is 350 ms or less, preferably wherein the predetermined time difference to the global maximum is 250 ms or less.

In a further aspect, the apparatus further comprises a light source configured for transmitting light into an extremity of a subject; wherein the means for providing pulse wave data comprises an optical sensor configured for receiving light reflected from blood flow through the extremity.

In a further aspect, the step of receiving the pulse wave data comprises activating the light source and receiving the pulse wave data based on a signal provided by the optical sensor.

In a further aspect, the optical sensor comprises a video sensor, and wherein the step of receiving the pulse wave data further comprises receiving a video stream indicative of the reflected light based on the signal; selecting a region of interest from the video stream containing a plurality of pixels, the region of interest optionally having a size of 50 x 50 pixels; selecting a plurality of frames from the video stream, each frame of the plurality of frames having a respective time stamp; for each respective frame: - determining, within the region of interest, a first sample value indicative of the sum of the values of a green subcomponent of each pixel of the plurality of pixels; - associating each first sample with the respective time stamp; - generating a first pulse wave from the first samples; and the step of receiving the pulse wave data further comprising determining a second pulse wave by re-sampling the first pulse wave based on the respective time stamps.

In a further aspect, determining the second pulse wave further comprises filtering the second pulse wave using a bandpass filter, the bandpass filter optionally removing all frequencies not falling within a range from 0.6 Hz to 2.5 Hz.

In a further aspect, the portion of the pulse wave data is indicative of 1 to 50 heart periods, preferably wherein the portion of the pulse wave data is indicative of 1 to 40 heart periods, more preferably wherein the portion of the pulse wave data is indicative of 10 to 30 heart periods.

In a further aspect, the portion of the pulse wave data is indicative of a plurality of successive heart periods.

In a further aspect, the sensor is an optical sensor and the apparatus further comprises a light source, the sensor being configured to detect a signal emitted by the light source and reflected by part of a body of the subject, optionally the part of the body of the subject comprising a pulsatile blood flow of the subject.

In a : further aspect, the apparatus further comprises input means configured to receive a user input initiating determining of the blood pressure value.

In a further aspect, the apparatus further comprises output means configured to display the blood pressure value.

In a further aspect, the means for providing pulse wave data comprises a memory unit configured to store the pulse wave data.

Advantages of the apparatus for determining the blood pressure include that the blood pressure can be determined with improved accuracy. The invention is set out in the appended claims.

## Brief description of drawings

FIG. 1 illustrates how the stiffness index is determined in accordance with the present invention;

FIG. 2 contains a flow chart of a method for determining blood pressure in accordance with a first embodiment of the invention;

FIG. 3A illustrates the detection of the respiratory rate in accordance with one embodiment of the invention;

FIG. 3B illustrates the relationship of the heart rate, blood pressure, and respiratory rate, as well as the variabilities thereof, in accordance with one embodiment of the invention;

FIG. 3C illustrates the application of the Shannon Entropy in detecting atrial fibrillation in a subject in accordance with one embodiment of the invention;

FIG. 4 contains a flow chart of a method for recording pulse wave data in accordance with the present invention, using a mobile device;

FIG. 5 illustrates an exemplary mobile device that can be used in accordance with the method of FIG. 4;

FIG. 5A illustrates an interaction of a human subject with the mobile device shown in FIG. 5;

FIG. 6 illustrates how a series of heart periods is determined based on acquired pulse wave data;

FIG. 7 illustrates how an exemplary adjustment function for adjusting the stiffness index to the age of a subject is determined;

FIG. 8 illustrates how an exemplary regression model for determining the blood pressure of a subject based on the adjusted stiffness index is determined;

FIGs. 9A and 9B illustrate the correlation of the respective blood pressure of a subject (as estimated based on the regression model and the alternative regression model) and the blood pressure of the subject measured using a common blood pressure monitor.

## Detailed Description

The elasticity of the vascular system influences the pulse wave of a subject. Based on this effect it has become possible to accurately determine (i.e. in the region of 90% accuracy or more) the blood pressure using an advanced form of photoplethysmography based on specific processing of the pulse wave data. Heart rate and respiratory rate can also be determine based on the pulse wave data of a subject.

The detailed analysis of each of these parameters can form the basis for determining individual conditions of a subject. However, it has been found that, given an accurate representation of the pulse wave data and taking measurements at regular intervals or continuously, the analysis of the heart rate (HR) and the heart rate variability, the blood pressure (BP) and the blood pressure variability, and the respiratory rate (RR) and the variability of the respiratory rate can serve to detect a range of medical conditions, such as CAD, AFIB, sleep apnea, depression and others.

The blood pressure and the blood pressure variability can be detected based on an advanced processing of pulse wave data and using the stiffness index.

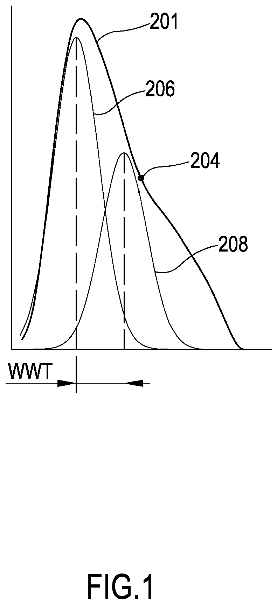

FIG. 1 illustrates how the stiffness index is determined in accordance with the present invention. The diagram in FIG. 1 shows a pulse wave signal 201 over time t as well as corresponding wave components 206 and 208 of the original pulse wave and the wave reflected mainly by the aortic bifurcation. FIG. 1 also shows an inflection point 204. It is noted that a simple partitioning based on the inflection points, as commonly known in the art, does not necessarily correspond to the actual physiological wave components because of the reasons set forth in the previous paragraph. In contrast, in accordance with the present invention, the actual original pulse wave and the wave reflected by the aortic bifurcation are determined by approximation of the graph with Gaussian functions, by which the two component waves w$_{original}$ 206 and w$_{reflected}$ 208 can be obtained with very high accuracy. Here, the time difference is determined as the time difference between the component waves w$_{original}$ and w$_{reflected}$ as opposed to the time difference between two maxima of the graph. This facilitates determining, instead of the commonly known peak-to-peak time (PPT), a wave-to-wave time (WWT), which corresponds to the actual time difference between the original pulse wave and the reflected pulse wave with a substantially higher level of accuracy. This, in turn, facilitates a more accurate calculation of the SI and, thus, leads to an improved correlation with the blood pressure.

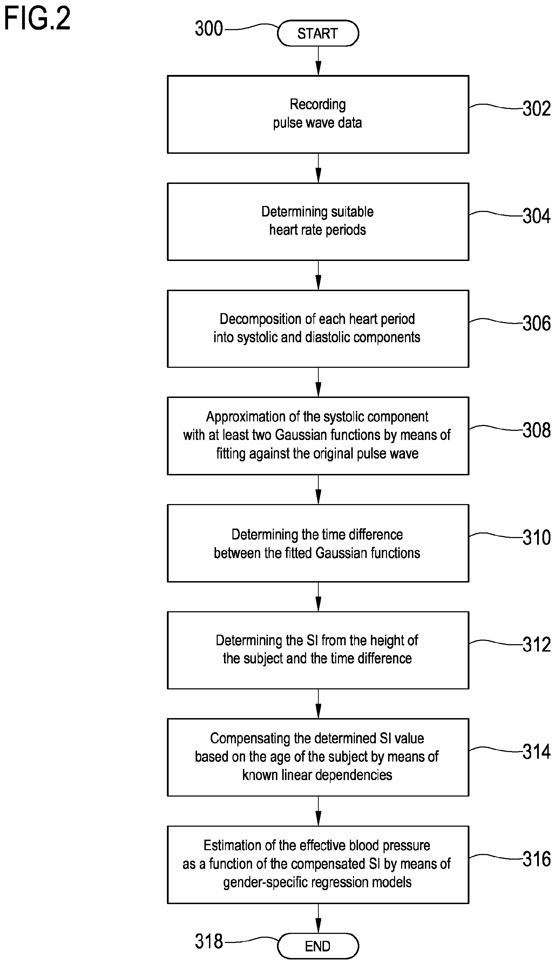

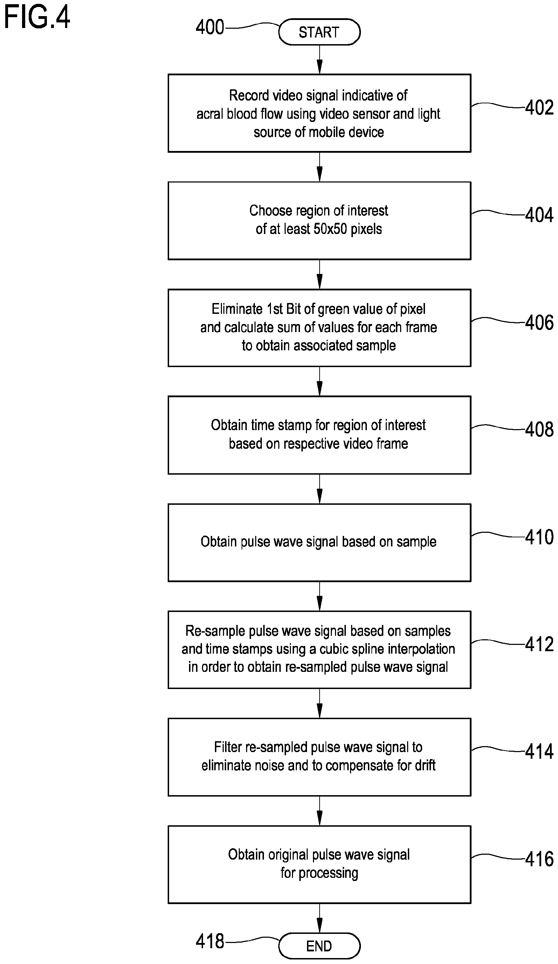

FIG. 2 contains a flow chart of a method 300 for determining blood pressure in accordance with a first embodiment of the invention. In step 302, pulse wave data is recorded. The detection of pulse waves and the recording of data indicative of the detected pulse wave can be performed in any way known in the art. For example, classic photoplethysmography. One example of detection and recording of pulse wave data is described further below with respect to FIG. 4.

In step 304 suitable heart periods are determined. As described above, heart periods vary depending on a number of factors and can exhibit benign (e.g. non-pathological) irregularities, for example caused by stress or anxiety, or consumption of stimulants such as caffeine, nicotine, or alcohol. In order to establish a sound basis for further processing of pulse wave data, suitable heart periods are selected from a longer recording of pule wave data. In the first embodiment, 5 to 30 heart periods are selected from a pulse wave recording of 5 seconds up to 2 minutes in length, provided that all selected heart periods have a similarity to each other of at least 0.8 and are all contained in a single recording segment (i.e. are successive to each other). In other embodiments, a greater or smaller number of successive heart periods may be used, for example 3 to 10 or 20 to 50 heart periods. Further, the recording of pulse wave data can have a different length, for example ranging from 5 to 10 seconds up to 10 to 30 minutes.

In step 306, each heart period is decomposed or partitioned into a systolic and a diastolic component. This is achieved by determining the maximum of the second order derivative of the pulse wave, located at most 350 ms after the systolic maximum. Typically, the maximum of the second order derivative of the pulse wave is located between 250 ms and 350 ms after the systolic maximum. Determining the maximum of the second order derivative is restricted to the above-defined time window in order to take into account the expulsion time of the heart and in order to avoid erroneous detection.

In step 308, an approximation is performed in which the systolic component is approximated by fitting at least two Gaussian functions to the original pulse wave:

$$F(a,b,c,d,f) = \sum_{i=1}^{N}\left( S_i - \left( a \cdot e^{-\frac{1}{2}\left(\frac{t-b}{c}\right)^2} + d \cdot e^{-\frac{1}{2}\left(\frac{t-f}{c}\right)^2} \right) \right)^2 \overset{!}{=} min$$

with a, *b,* c, and d being determined using non-linear optimization. In one embodiment, the two Gaussian functions are fitted to the original pulse wave using the Levenberg-Marquardt algorithm. In this approximation step, the first Gaussian function corresponds to the original pulse wave and the second Gaussian function corresponds to the wave reflected at the aortic bifurcation, whereas the amplitude of the first Gaussian function must be greater or equal to the amplitude of the first Gaussian function, and both functions must exhibit an identical standard deviation σ.

In step 310, the time difference between the two Gaussian functions is calculated as the wave-to-wave time WWT. For example, the WWT can be calculated as the time difference between the base points of the two Gaussian functions. Alternatively, the WWT can be calculated as the time difference between the maxima of the two Gaussian functions. In order to generate an overall or averaged WWT$_a$, the median value over 5 to 30 (or any desired number of) heart periods is calculated. This can effectively reduce the impact of outliers.

In step 312, the stiffness index SI is calculated based on the subject height h (in m) and the averaged WWT$_a$ (in s) as:

$$SI = \frac{h}{WWT_a}$$

In step 314, the SI value calculated in step 312 is adjusted in order to compensate for the age of the subject. As described above, the elasticity of a person's vascular system decreases with increasing age, so that the average healthy person at an age of 20 necessarily exhibits a lower SI than the average healthy person at an age of 40 or 60. Therefore, the SI is normalized in step 314 in order to achieve comparable results. In the first embodiment, the SI is normalized in order to obtain an age-independent or adjusted SI.

In step 316, the adjusted SI is estimated based on a gender-specific regression model. The gender-specific regression models are the result of proprietary clinical studies and define the estimated blood pressure of a subject as a function of gender and the adjusted SI. In one example, a male person exhibiting an adjusted SI of 10 may have an estimated systolic blood pressure of 180 mm Hg. Clinical studies were conducted in order to determine how the adjusted SI relates to the actual blood pressure depending on the gender of a subject. It has been found that, with male subjects, an adjusted SI of about 10 m/s corresponds to a blood pressure of about 170 mm Hg, and an adjusted SI of about 8 m/s corresponds to a blood pressure of about 150 mm Hg. With female subjects, an adjusted SI of about 10 m/s corresponds to a blood pressure of about 165 mm Hg, and an adjusted SI of about 8 m/s corresponds to a blood pressure of about 155 mm Hg.

In an alternative embodiment, a more comprehensive regression model is applied. In this alternative embodiment, steps 302 to 314 are performed identical to what is described above. In step 316 of the alternative embodiment, however, additional parameters are applied in order to achieve an even higher correlation to the actual blood pressure value. Here, the adjusted SI is estimated based on an alternative regression model that factors in: the gender of the subject (i.e. male or female), an index value $I_f$ indicative of the physique of the subject (e.g. the body mass index (BMI) of the person), and an index value Ct indicative of a tobacco consumption of the subject (e.g. whether or not the subject is a smoker).

With respect to the index value Ct indicative of a tobacco consumption of the subject it is noted that in some embodiments only the current status of a subject is determined, namely whether the subject is currently an active smoker. Studies have shown that a relatively short period of not smoking has an impact on blood pressure in a subject, even if the subject had smoked for an extensive period of time. This effect and related effects can be taken into account by determining the current status of a subject in this manner. In other embodiments the history of the subject can also be taken into account. This can be done by determining a period or periods in which the subject was an active smoker and determining the amount of tobacco consumed in these periods (e.g. number of cigarettes per day). In this manner an individual profile detailing the consumption of tobacco by a subject can be generated and introduced into the regression model. It is noted that long-term tobacco consumption can have multiple effects on the vascular system of a subject, for example regarding stiffness of the blood vessels. Some or all of these effects can be long-term effects that do not disappear during a short period of non-smoking.

One specific alternative regression model, which is also the result of proprietary clinical studies, defines the estimated blood pressure of a subject as a function of the adjusted SI, the gender of the subject (a value of 1 being indicative of a male subject, a value of 2 being indicative of a female subject), the BMI of the subject (the BMI value being calculated based on the height and weight of the subject), and the fact that the subject is a smoker or not (a value of 0 being indicative of the subject not being a smoker, and a value of 1 being indicative of the subject being a smoker).

The BMI can be calculated based on $BMI = \frac{mass}{height^2}$, where mass is the weight of the subject in kilograms (kg) and where height is the height of the subject in meters (m). The specific alternative regression model is based on the formula: $BP_{sys}$ = 139.611 - 19.450 · $g$ - 0.820 · $age$ + 0.968 · $I_f$ + 5.394 · $C_t$ + 2.759 · $SI$.

The following table provides further details on the coefficients used in the alternative regression model:

Coefficients (dep. var: $BP_{sys}$)

| Model | | Non Standardized Coefficients | | Standardized Coefficients | t | Sig. |
|---|---|---|---|---|---|---|
| | | B | Standard Error | Beta | | |
| 1 | (Constant) | 139.611 | 40.618 | | 3.437 | 0.004 |
| | Sex (1=male, 2=female) | -19,450 | 7.278 | -0.568 | -2.673 | 0.019 |
| | Age | -0.820 | 0.478 | -0.318 | -1.717 | 0.110 |
| | $I_f$ (BMI) | 0.968 | 1.513 | 0.140 | 0.639 | 0.534 |
| | $C_t$ (Smoker) | 5.394 | 6.830 | 0.144 | 0.790 | 0.444 |
| | SI | 2.759 | 2.286 | 0.228 | 1.207 | 0.249 |

It is noted that the term "physique" of the subject refers to the size, stature, figure, or physique in terms of the absence or presence (and the degree) of adiposity of the subject, i.e. whether the person is overweight or not. Apart from the BMI as described above, there are a number of known methods and/or concepts for quantifying a degree of adiposity in a subject. Examples include, but are not limited to: measuring the percentage of body fat (e.g. by bioelectric impedance analysis, by caliper measurements, or any other known method for determining the percentage of body fat), calculating the waist-to-height ratio, and calculating the waist-to-hip ratio. Bioelectric impedance analysis, for example, can be integrated into household appliances like scales, so that the percentage of body fat can be measured during regular or daily activities, such as stepping on the scale to be weighed. Bioelectric impedance analysis may not be applicable to all subjects due to their individual medical condition, for example when a pace maker or other implant is in place, and/or may not provide the most accurate measurements of body fat percentage. Caliper measurements can be made by a physician or by the subject him/herself by measuring the thickness of a skin fold in order to deduce the body fat percentage. The measurements are typically performed at three or seven different body parts, depending on the method used. Caliper measurements can provide acceptable results but typically cannot accurately measure the percentage of body fat present in and around organs.

It is noted that the alternative regression function described above does not require the use of the BMI in particular, but is, in principle, adaptable to any quantification of a degree of adiposity in a subject. If a measure of a subject's physique other than the subject's BMI is used, a corresponding conversion factor needs to be introduced into the specific formula described above, in order to map the measure to the BMI (or vice versa).

Blood pressure variability is now determined based on a plurality of blood pressure values taken from a subject in the manner described above. Typically, determining blood pressure variability is performed over a period of 2 to 5 minutes, or alternatively, over a number of 120 to 300 heart periods, in order to obtain a representative sample. In other embodiments, however, the determining of blood pressure and blood pressure variability can be performed in a continuous manner, for example using a sliding window of 2 to 5 minutes (or 120 to 300 heart periods).

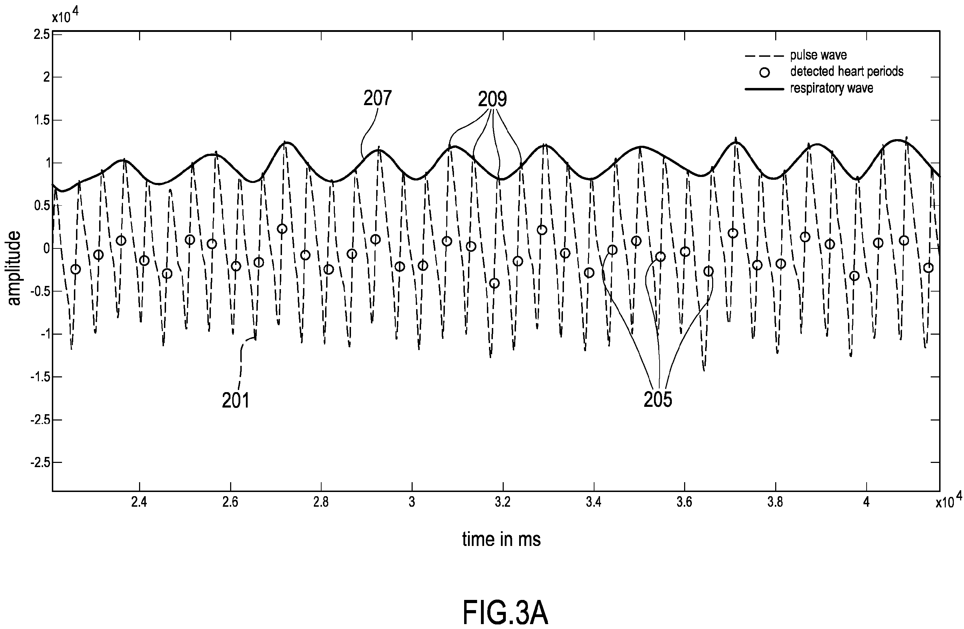

FIG. 3A illustrates the detection of the respiratory rate in accordance with one embodiment of the invention. FIG 3A shows, on the vertical axis, the amplitude a detected pulse wave 201 over time (see horizontal axis). The pulse wave 201 has an amplitude of between approximately -1 and 1, and the instances of rising edges are registered as detected heart periods 205. Further, a signal 207 indicative of the respiration of the subject is detected based on the maxima 209 of the pulse wave 201. In order to obtain the signal, the maxima 209 of the pulse wave are sampled using a cubic spline interpolation similar to the re-sampling of the pulse-wave described with respect to FIG. 4 below. Here, two subsequent samples are interpolated by a third-degree polynomial. The position (in time) of the samples corresponds to the time stamps. The polynomial $R_i$ for the range $[t_i, t_{i+1}]$ is calculated as follows:

$$R_i = a_i + b_i(t - t_i)^2 + d_i(t - t_i)^3$$

with $i = 1, ..., n$-1. The process of re-sampling includes incrementing t continuously by 1 ms, corresponding to a sample rate of 1000 Hz. The parameters $a_i$, $b_i$, $c_i$, and $d_i$ have to be set to suitable values. The pulse wave is obtained as the respiration R, i.e. signal 207, being the result of the sampling. The variation of the respiration rate is then determined based on signal 207 by known methods, for example by detecting a series of maxima of signal 207 and determining a time difference for each pair of subsequent maxima.

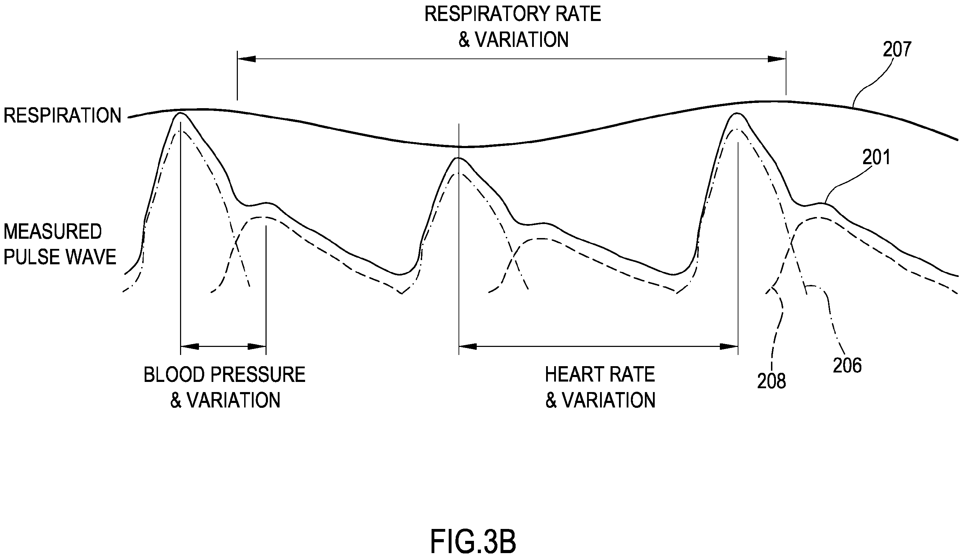

FIG. 3B illustrates the relationship of the heart rate, blood pressure, and respiratory rate, as well as the variabilities thereof, in accordance with one embodiment of the invention. FIG. 3B shows a combination of a number of signals determined based on the pulse wave 201. Here, the respiratory rate and the variation thereof are shown based on signal 207. Further, the blood pressure and variation thereof are shown based on pulse wave 201 and the components 206 and 208 thereof, as described above and as shown in FIG. 1. The heart rate and variation thereof is also shown based on pulse wave 201.

Based on an analysis of the heart rate (HR) and the heart rate variability, the blood pressure (BP) and the blood pressure variability, and the respiratory rate (RR) and the variability of the respiratory rate, all obtained based on the pulse wave 201 and exhibiting an accuracy previously not obtainable, a range of medical conditions, such as CAD, AFIB, sleep apnea, depression and others.

Based on the data obtained, AFIB can be detected by analyzing the interaction between heart rate and blood pressure using nonlinear interaction dynamics, for example joint symbolic dynamics (JSD) and segmented Poincare plot analysis (SPPA). SPPA can be applied to analyze the interaction between two time series - here heart rate and blood pressure. Introducing a parameter set of two indices, one derived from JSD and one from SPPA, the linear discriminant function analysis revealed an overall accuracy of 89% (sensitivity 91.7%, specificity 86.7%) for the classification between patients with stable sinus rhythm (group SR, n = 15) and with AF recurrence (group REZ, n = 12). The coupling of heart rate and systolic blood pressure provides a potential tool for the prediction of AF recurrence after CV and could aid in the adjustment of therapeutic options for patients with AF.

In a similar manner, depression can be detected by analyzing the relationship between respiration and heart rate and by detecting that respiration and heart rate are not in sync and/or the heart rate does not change upon substantial variation of the respiratory rate. Likewise, sleep apnea can be detected using the above-described mechanisms by analyzing the respiratory rate, typically showing an unusually high variation, and by analyzing the heart rate, typically slowing down during periods of sleep apnea.

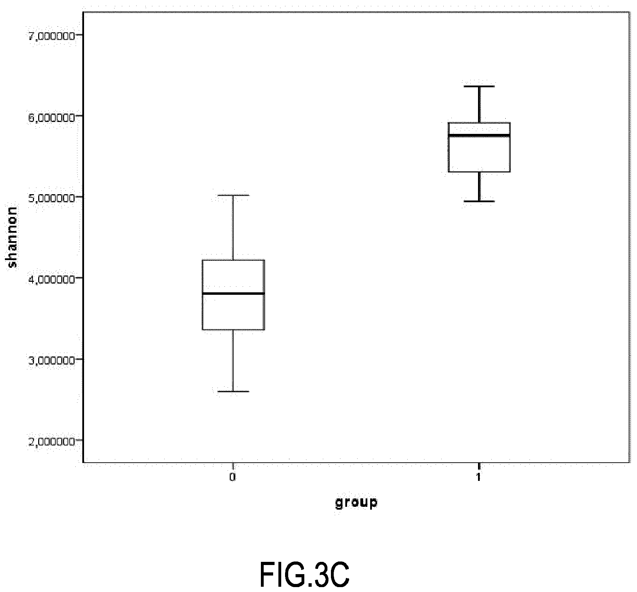

FIG. 3C illustrates the application of the Shannon Entropy in detecting atrial fibrillation in a subject in accordance with one embodiment of the invention. Based on the pulse wave, a tachogram is determined, which is indicative of the variations in respiratory rate over time. From the tachogram, a histogram is generated, which represents the frequency distribution of the respiratory rate variations. In one embodiment, the histogram has a bin size of 8ms, which means that the frequency distribution is based on a discrete time scale divided into 8ms slots. Each respiratory variation (i.e. between two maxima of signal 207) is sorted into the respective bin. The probabilities represented by the histogram are then used as input for calculating the Shannon Entropy as

$$S = -\sum_{i=1} p_i \cdot log_2(p_i) \ .$$

The result is a bit value, which determines whether or not a subject belongs to a group of healthy patients or not, whereas a threshold value of 4.8 bits is used:

$$AFib = \begin{cases} S \geq 4{,}8 \ bit, \ then \ \textbf{yes} \\ otherwise \ \textbf{no} \end{cases}$$

It is noted that the above is one example to determining an entropy value for the respiratory rate variations. Other known methods can be used in a similar manner, by simply adapting the threshold value according to the method and calculation used. FIG. 3C illustrates the threshold value of 4.8, clearly distinguishing between subjects showing AFIB (right; value "1") and not showing AFIB (left; value "0"). One advantage of determining a frequency distribution in the manner described is that the entropy measure is independent from a resting heart rate of the subject. The above is, thus, equally applicable to subjects of all age groups, for example children as well as the elderly, despite of substantial differences in their respective resting heart rates.

FIG. 4 contains a flow chart of a method 400 for recording pulse wave data in accordance with the present invention, using a mobile device having video recording capabilities. Mobile communication devices, in particular so-called smart phones, have extensive capabilities beyond mere telecommunication. For example, most mobile phones are typically provided with a digital camera capable of capturing still images and video and with a corresponding light source for low-light situations. In general, to record a pulse wave by detecting, with an optical sensor, light emitted from a light source and reflected by a finger of a subject. In one embodiment, pulse wave data is obtained using a common mobile device equipped with a digital camera (e.g. used as an optical sensor) and an LED flashlight (e.g. used as a light source). The light emitted by the light source is reflected and the properties of the light (e.g. intensity, hue, brightness, saturation) are affected (e.g. one or more of the properties are modulated) by the acral blood flow.

In step 402, the subject places their finger on both the light source and the camera of the mobile device such that light emitted from the light source illuminates the acral blood flow and is reflected and detected by the camera. The video signal thus created is recorded and stored in a memory unit of the device. Alternatively, the video signal (e.g. a video stream) can be processed directly, without necessitating storing the pulse wave data in a memory unit.

In step 404, a region of interest (ROI) is selected from the full resolution video stream. This selection can be performed, for example, based on brightness information contained in the video stream. In one embodiment, the ROI is determined in a region of maximum brightness within a video frame, off the center and at a minimum distance from the border. This can ensure that a region is chosen that is sufficiently illuminated (e.g. a region close to the light source). In one embodiment, the ROI has a size of at least 50 x 50 pixels (i.e. 2500 square pixels). Generally, the ROI can have a size ranging from 625 to 10000 square pixels, preferably 900 to 6400 square pixels, more preferably 1600 to 3200 square pixels.

In step 406, for the ROI of each frame of the video stream, a sample $s_i$ is calculated, based on

$$s_i = \sum_{j=0}^{N-1} \sum_{k=0}^{M-1} \frac{p(j \cdot w + k)}{2}$$

with $p$ being the value of the green channel of the pixel located within the ROI at the position $j,k$; N and M being the size of the ROI; and w being the width of the ROI. The division by 2 eliminates the lowest Bit of $p$, such that noise is effectively reduced. This produces a sample $s_i$ for each captured video frame.

In step 408, a time stamp $t_i$ is generated for each sample $s_i$ (more accurately, for each video frame, based on which the sample was calculated) and encoded into the video stream by the video camera.

In step 410, the pulse wave is obtained as a pulse wave signal based on the samples $s_i$ obtained in step 406.

In step 412, a re-sampled pulse wave is obtained by re-sampling the pulse wave from the samples $s_i$ (i.e. as obtained in step 410) based on the associated time stamps obtained in step 408. This is necessary due to technical issues in detecting, generating, and encoding video data, for example resulting in dropped frames or non-constant frame rates. Based on these issues, the samples $s_i$ cannot be obtained at fixed and reliable time intervals. In order to obtain the re-sampled pulse wave, the pulse wave is re-sampled using a cubic spline interpolation and is performed on each polynomial. Here, two subsequent samples are interpolated by a third-degree polynomial. The position (in time) of the samples corresponds to the time stamps. The polynomial $S_i$ for the range $[t_i, t_{i+1}]$ is calculated as follows:

$$S_i = a_i + b_i(t - t_i)^2 + d_i(t - t_i)^3$$

with $i$ = 1, ..., $n$-1. The process of re-sampling includes incrementing t continuously by 1 ms, corresponding to a sample rate of 1000 Hz. The parameters $a_i$, $b_i$, $c_i$, and $d_i$ have to be set to suitable values. The pulse wave is obtained as the signal S being the result of the re-sampling.

In step 414, the re-sampled pulse wave is filtered to eliminate noise and to compensate for drift. This can be achieved by applying a common bandpass filter (e.g. 0.1 to 10 Hz).

In step 416, the original pulse wave signal is obtained in order to be processed further, as described above with respect to FIG. 3 (see, e.g., steps 304 ff.)

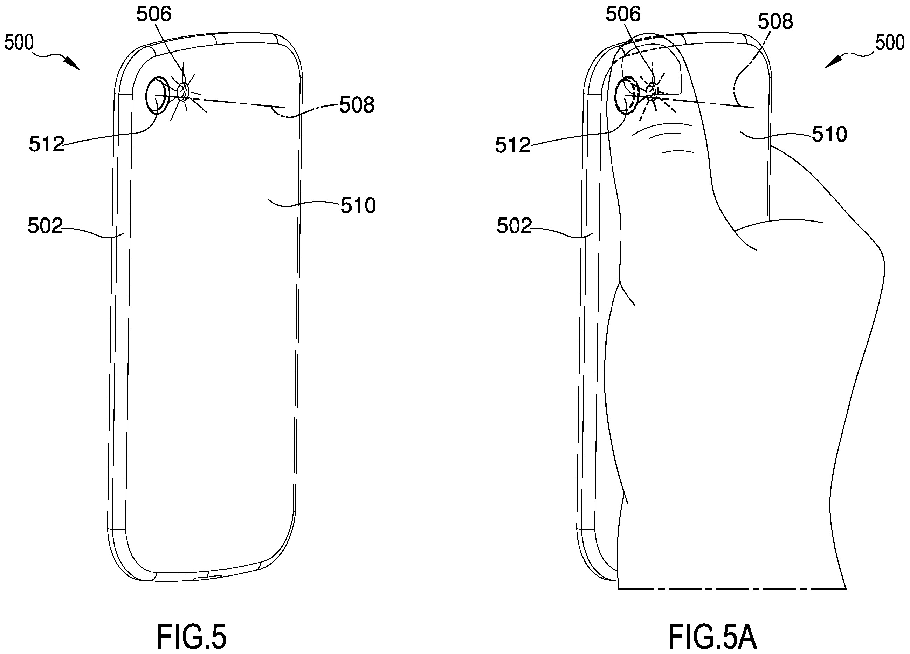

FIG. 5 illustrates a exemplary mobile device that can be used in accordance with the method of FIG. 4. The mobile device 500 has a frame or main body 502 and a device panel 510. In some examples, the device panel 510 can be a back panel of the mobile device 500. The device 500 further has a camera device 512 capable of detecting digital video signals, for example in the form of digital still images and digital video. The camera device 512 is configured to detect video signals representative of objects located generally with a frustum-shaped region along a main detection direction 508. The device 500 further has a light source 506 configured to illuminate any objects located in front of camera device 512, i.e. located within the frustum-shaped region and/or along a main detection direction 508. The light source 506 can be configured to provide both a single flash of light and a continuous light beam, depending on a mode of operation. When recording video, the light source typically provides a continuous light beam. Light emitted from light source 506 will be reflected by an object placed within the view of camera device 512 so that the reflected light can be detected by camera device 512. Mobile device 500 further comprises a control unit (e.g. a CPU, micro processor, SoC; not shown) coupled to other components, such as camera device 512, light source 506, a memory unit, a user interface, input means, an audio unit, a video unit, a display, and other.

FIG. 5A illustrates an interaction of a human subject with the mobile device shown in FIG. 5. In order to take a measurement, the subject places a finger (e.g. a thumb) on mobile device 500, covering both the camera device 512 and the light source 506. The individual configuration of the mobile device (e.g. a position of camera device 512 and light source 506 or the distance in between) is of secondary relevance, as long as it is physically possible to cover both the camera device 512 and the light source 506 with a suitable extremity (e.g. finger, thumb, ear). In this respect, any extremity suitable for (acral) measurement can be used in accordance with the present invention. In general, any body part that is associated with pulsating blood flow can be used in accordance with the present invention, as long as a meaningful signal indicative of the blood flow can be detected via the body part. In some embodiments, the control unit of mobile device 500 will process signals provided by camera device 512 and detect, based on the signals provided, that one or more parameters indicative of video quality (e.g. brightness, contrast, focus) are outside of preferred operating ranges due to the low-light and/or close-proximity situation created by the placement of the thumb directly onto camera device 512. The control unit may then provide control signals to one or more components, for example to light source 506, in order to make adjustments to the parameters (e.g. activating light source 506 in order to compensate for low light).

Upon placement of the suitable extremity (here, e.g., the thumb of the subject), the measurement is initiated by activating the light source 506 to emit a continuous light beam of sufficient intensity, such that acral blood flow is illuminated. At substantially the same time, camera device 512 is activated and the light reflected by the acral blood flow is detected by camera device 512. Both activating the light source 506 and activating the camera device 512 can be achieved by corresponding program code executed by the control unit comprised in device 500. The activation can be triggered manually, for example by selecting a corresponding function on a user interface of device 500, or automatically, for example triggered by a sensor (e.g. a proximity sensor, an optical sensor), a timer, voice recognition, or other (input means). In one example, the signal of the sensor is continuously processed to check for the presence of a suitable signal. Video data is then recorded or transmitted for further processing for a predetermined period of time, typically ranging from several seconds to 2 minutes. In some embodiments, the time period is not predetermined, but determined as the recording/transmitting is ongoing, in that a quality measure is calculated from the recorded/transmitted data and the recording/transmitting is performed until a sufficient number of heart periods (e.g. 10-30) of sufficient quality (e.g. similarity; see in further detail below) has been recorded/transmitted. Completion of the recording/transmitting can be indicated to the subject, for example, by an acoustic and/or optical signal emitted by an audio and/or video component of device 500.

It is noted that other embodiments employ the same or different sensors and/or devices. For example, smart watches having a corresponding light source/sensor assembly as described above with respect to FIGs. 5 and 5A, can be used as well. These devices have an advantage in that the sensor is kept in close proximity to the body (here: wrist) of a subject, thereby facilitating continuous measurements and/or measurements of arbitrary duration and at arbitrary time points, without interaction of a subject (e.g. also during sleep). It is noted that the above concepts apply to a range of sensors and are not limited to a particular or otherwise specific embodiment of sensor hardware.

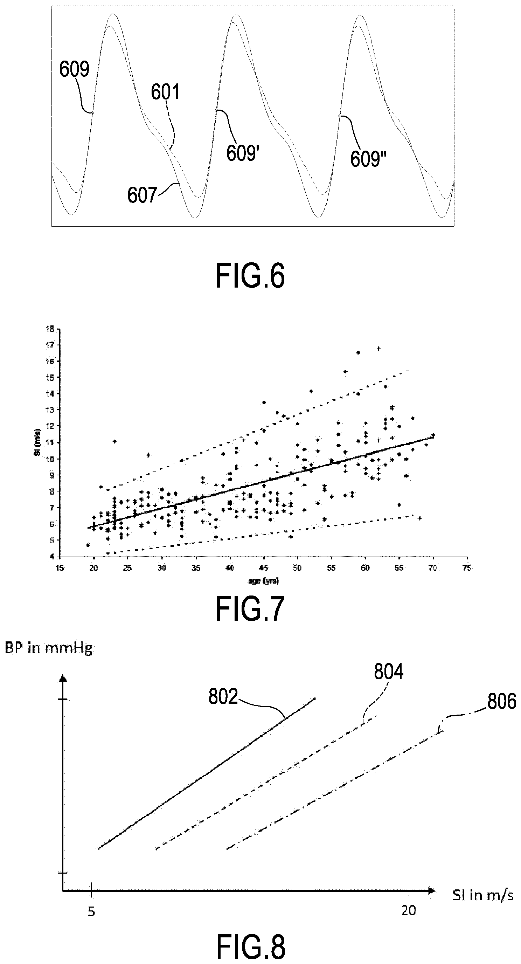

FIG. 6 illustrates how a series of heart periods is determined based on acquired pulse wave data 601. Pulse wave data can be acquired from live measurements taken with a human subject or can be retrieved from data storage when measurements recorded at an earlier time are to be processed. Pulse wave data 601 contains signals corresponding to a number of heart periods exhibited by the subject over an extended time period. In some examples, the pulse wave data cover several minutes of recorded heart periods, for example 2 minutes, preferably several seconds to 2 minutes. In other examples, the pulse wave data can cover substantially less (e.g. 5-30 seconds) or more (several hours) of recorded heart periods. For reasons of clarity, FIG. 6 shows merely three successive heart periods representing only a small window of pulse wave data covering an extended period of time of typically up to 2 minutes.

The pulse wave data 601 is partitioned into single heart periods by generating an amplified wanted signal 607 from the original pulse wave 601 and scanning the amplified signal for rising edges. In general, a pulse wave comprising a single heart period is sufficient, but typically a pulse wave comprising a plurality of successive heart periods is used. In detail, a spectrum is created from the filtered (see FIG. 4, step 414, and corresponding description above) pulse wave signal 601 using discrete Fourier transformation (DFT): $Spec = |DFT(S_{filter})|$. In this spectrum, the maximum frequency in the range of 0.6 Hz to 2.5 Hz is determined and regarded as the dominant heart frequency: $idx = argmax\{Spec_{range}\}$, wherein $Spec_{range}$ corresponds to the spectrum from 0.6 Hz to 2.5 Hz and $idx$ corresponds to the index (i.e. frequency) in the spectrum. Then, a normalized Gaussian graph having values in the range [0,1] is superposed over the dominant heart frequency and over the 2 harmonic components thereof, such that a minor variation of the heart rate is accounted for. The standard deviation $\sigma$ of the Gaussian graphs should intersect at $3\sigma$, with: $\sigma = \frac{idx}{6}$ and

$$gauss(t) = e^{-\frac{1}{2}\left(\frac{t-idx}{\sigma}\right)^2} + e^{-\frac{1}{2}\left(\frac{t-2\cdot idx}{\sigma}\right)^2} + e^{-\frac{1}{2}\left(\frac{t-3\cdot idx}{\sigma}\right)^2}.$$ The wanted signal is obtained by multiplying the spectrum with the Gaussian function and subsequent back transformation: $S_{wanted} = Real(IDFT(spec \cdot gauss))$. The amplified signal $S_{amp}$ is then obtained by multiplication of the wanted signal and addition to the original signal:

$$S_{amp} = \frac{1}{2}\left(\frac{S_{filter}}{f} + f \cdot S_{nutz}\right),$$ with $f$ being the amplification factor. Subsquently, the first order derivative of the amplified signal $S_{amp}$ is calculated and the maxima thereof, indicating the inflection points on the amplified signal $S_{amp}$, and, thus, the rising edges thereof. This provides the location of each heart period, defined between the two local minima before and after the rising edge of each heart period.

For a successive number of heart periods, a similarity score is then determined. A cross correlation of each heart period with a template heart period $P_{template}$ is calculated and a predetermined number of heart periods (e.g. 10 heart periods) having the highest correlation is obtained. In one embodiment, the similarity (i.e. correlation) of successive heart periods is 0.9 or greater. If each heart period of a minimum number of successive heart periods (e.g. 10-30) fulfills the similarity requirement, then a portion of the pulse wave suitable for further processing has been identified.

FIG. 7 illustrates how an exemplary adjustment function for adjusting the stiffness index to the age of a subject is determined. The horizontal axis of the graph indicates the age of a subject (in years) and the vertical axis indicates the SI (in m/s). The distribution of measured SI of a number of subjects and a correlation with the age of the respective subject provides a statistical basis for computing the adjustment function as shown in FIG. 7. Here, the SI of a subject being 60 or 65 years of age can be correlated to the SI of a subject being 20 or 25 years of age.

FIG. 8 illustrates how an exemplary regression model for determining the blood pressure of a subject based on the adjusted stiffness index is determined. The regression model is age-dependent in that regression line 802 serves to provide a regression function for subjects aged 20 to 30 years. In the same manner, regression lines 804 and 806 serve to provide regression functions respectively for subjects aged 30 to 40 years and 60 to 70 years. The regression model facilitates associating the SI of a subject belonging to a particular age group to a corresponding blood pressure value. As the data underlying the regression model is updated, the regression model can be adjusted over time in order to improve the accuracy thereof.

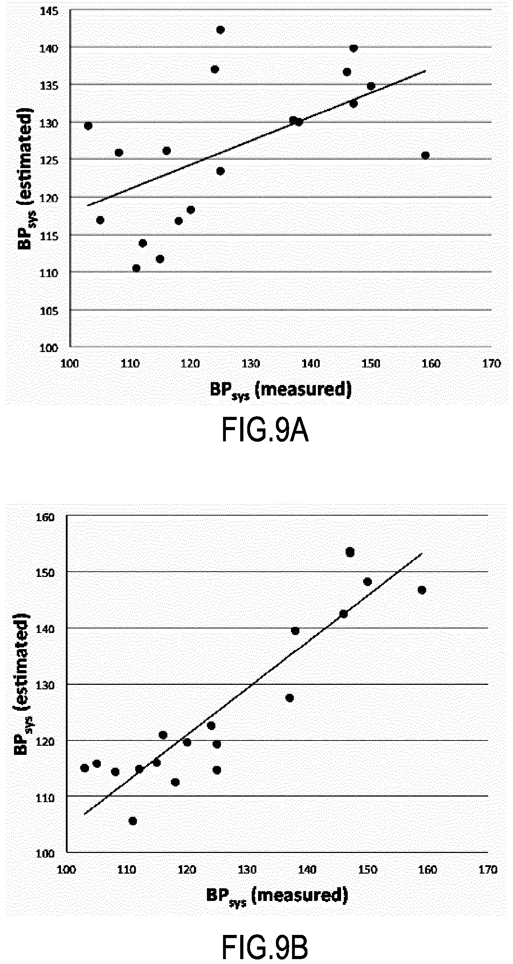

FIGs. 9A and 9B illustrate the correlation of the respective blood pressure of a subject (as estimated based on the regression model and the alternative regression model) and the blood pressure of the subject measured using a common blood pressure monitor. FIG. 9A illustrates the correlation of the estimated blood pressure of a subject and the blood pressure of the subject measured using a common blood pressure monitor. The blood pressure was estimated based on the regression model described above and the correlation was R=0.57. FIG. 9B illustrates the correlation of the estimated blood pressure of a subject and the blood pressure of the subject measured using a common blood pressure monitor. The blood pressure was estimated based on the alternative regression model described above and the correlation was R=0.91.

**Claims**

1. An apparatus for determining a medical condition of a human subject, the apparatus comprising:

   a control unit; and
   a means for providing pulse wave data representative of a heart beat of the human subject; wherein
   the control unit is configured to perform the steps of:

   receiving the pulse wave data;
   selecting a portion of the pulse wave data indicative of a plurality of heart periods;
   for the portion of the pulse wave data indicative of a plurality of heart periods:

   - determining a blood pressure variability based on the pulse wave data of the portion of the pulse wave data indicative of a plurality of heart periods;
   - determining a respiratory rate variability based on the pulse wave data of the portion of the pulse wave data indicative of a plurality of heart periods; and
   - determining a heart rate variability based on the pulse wave data of the portion of the pulse wave data indicative of a plurality of heart periods; and

   determining at least one correlation value based on at least one of the blood pressure variability, the respiratory rate variability, the heart rate variability, and a respective reference value; and
   determining a medical condition of the subject based on the at least one correlation value; wherein

   the subject has a body height, an age, and a gender, and
   the step of determining the blood pressure variability comprises determining a plurality of blood pressure values, the step of determining a plurality of blood pressure values comprising, for each respective blood pressure value of the plurality of blood pressure values, each respective blood pressure value being associated with a respective heart period of the plurality of heart periods:

   - determining a systolic component of the respective heart period;
   - approximating the systolic component with a first Gaussian function and a second Gaussian function; and
   - determining a time difference (WWT) between the first and second Gaussian functions; and

   determining a respective blood pressure value (BP) of the plurality of blood pressure values of the subject based on the time difference (WWT), the body height, and/or the age; and wherein
   the step of determining a plurality of blood pressure values further comprises, for each blood pressure value of the plurality of blood pressure values:

   - determining a preliminary stiffness index ($SI_p$) based on the body height and the time difference (WWT);
   - determining an adjusted stiffness index ($SI_a$) based on the preliminary stiffness index ($SI_p$) and the age; and
   - determining the blood pressure value (BP) based on the adjusted stiffness index ($SI_a$) and a regression model.

2. The apparatus according to the preceding claim, wherein the pulse wave data indicative of a plurality of heart periods relates to a plurality of heart periods in direct succession to one another.

3. The apparatus according to any one of the preceding claims, wherein the step of determining the respiratory rate variability based on the pulse wave data of the portion of the pulse wave data indicative of a plurality of heart periods comprises:

   determining a plurality of maxima based on the pulse wave data, the plurality of maxima denoting the maximum amplitude of a respective plurality of heart periods;
   determining a respiratory signal indicative of the respiratory rate based on the plurality of maxima, optionally including determining the respiratory signal based on a spline interpolation of the plurality of maxima; and determining the respiratory rate variability based on a time difference between each maximum of the respiratory signal.

4. The apparatus according to any one of the preceding claims, wherein the step of determining the heart rate variability based on the pulse wave data of the portion of the pulse wave data indicative of a plurality of heart periods comprises:

determining a plurality of reference points based on the pulse wave data, the plurality of reference points corresponding to a respective component of the plurality of heart periods, optionally the respective component being one of a maximum amplitude of the heart period, a rising edge of the heart rate amplitude;
determining the heart rate variability based on a time difference between each reference point of the plurality of reference points.

5. The apparatus according to any one of the preceding claims, wherein

- the portion of the pulse wave data is indicative of a plurality of heart periods, and wherein the step of determining the time difference (WWT) further comprises:
determining the time difference (WWT) for the plurality of successive heart periods as an average value based on the respective time differences determined for the plurality of heart periods; optionally the average value being the median value of the determined respective time differences; and/or wherein
- the first and second Gaussian functions have a respective maximum amplitude, the maximum amplitude of the first Gaussian function being greater than or equal to the maximum amplitude of the second Gaussian function; and/or wherein
- the first and second Gaussian functions have respective first and second standard deviations ($\sigma_1$, $\sigma_2$), the first and second standard deviations ($\sigma_1$, $\sigma_2$) being equal to each other.

6. The apparatus according to any one of the preceding claims, wherein the step of approximating the systolic component comprises: fitting the first and second Gaussian functions to the systolic component using

$$F(a,b,c,d,f) = \sum_{i=1}^{N}\left( S_i - \left( a \cdot e^{-\frac{1}{2}\left(\frac{t-b}{c}\right)^2} + d \cdot e^{-\frac{1}{2}\left(\frac{t-f}{c}\right)^2} \right) \right)^2 \overset{!}{=} min$$

with *a, b, c,* and *d* being determined using non-linear optimization or curve-fitting.

7. The apparatus according to any one of the preceding claims, wherein the regression model comprises a regression function

$$f(SI_a, g) = BP_{sys},$$

where $SI_a$ is the adjusted stiffness index ($SI_a$), g is the gender of the subject, and $BP_{sys}$ is the blood pressure; and wherein determining the blood pressure value comprises determining the blood pressure value based on the regression function, wherein the regression function comprises a linear function of the type

$$f(x) = ax + b,$$

wherein a ranges from 1 to 20 mmHg/(m/s) and *b* ranges from 0 to 80 mmHg, more preferably wherein *a* ranges from 5 to 15 mmHg/(m/s) and *b* ranges from 20 to 60 mmHg.

8. The apparatus according to any one of the preceding claims, wherein determining the adjusted stiffness index ($SI_a$) is based on an adjustment function

$$f(SI_p) = SI_a,$$

where $SI_p$ is the preliminary stiffness index and $SI_a$ is the adjusted stiffness index ($SI_a$), optionally wherein the adjustment function is a linear function of the type

$$f(x) = cx + d,$$

where $c$ and $d$ are adjustment factors determined based on a plurality of value pairs comprising an age value and an associated stiffness index value; optionally wherein:

$$c = \frac{SI - \mu}{range(age)}$$

with $\mu = 0{,}109 * age + 3{,}699$ and $range(age) = 0{,}1663 * age + 4{,}3858 - \mu$, $age$ being the age of the subject, and $d = 0$.

9. The apparatus according to any one of the preceding claims, wherein determining the systolic component comprises:

   determining a respective global maximum of the respective heart period;
   determining the second order derivative of the respective heart period;
   determining a maximum value of the second order derivative located at least at a predetermined time difference from the global maximum; and
   defining the systolic component as a portion of the heart period between the start of the heart period and the maximum value; optionally the predetermined time difference to the global maximum being 350 ms or less, further optionally the predetermined time difference to the global maximum being 250 ms or less.

10. The apparatus according to any one of the preceding claims, wherein determining the preliminary stiffness index ($SI_p$) is based on a function

$$SI_p = \frac{h}{WWT},$$

where $h$ is the subject height, $WWT$ is the time difference, and $SI_p$ is the preliminary stiffness index ($SI_p$).

11. The apparatus according to any one of the preceding claims, wherein the step of determining at least one correlation value is based on the heart rate variability; the step of determining at least one correlation value further comprising:

   generating, based on a plurality of heart rate variability values, a frequency distribution indicative of the distribution of the plurality of heart rate variability values in the time domain;
   determining a plurality of expected values;
   determining an entropy value indicative of a plurality of expected values, the entropy value being indicative of the medical condition of the subject.

12. The apparatus according to the preceding claim, wherein the frequency distribution indicative of the distribution of the plurality of heart rate variability values comprises a histogram, optionally wherein the histogram has a bin size of 8 ms.

13. The apparatus according to any one of the preceding claims, wherein the portion of the pulse wave data indicative of a plurality of heart periods covers a period of between 2 minutes and 5 minutes; and
   the step of determining the variabilities of the blood pressure, respiratory rate, and the heart rate variability, is based on substantially all heart beats comprised in the pulse wave data.

14. The apparatus according to any one of the preceding claims, wherein the step of determining at least one correlation value is based on the heart rate variability and the respiration rate variability, and wherein the step of determining at least one correlation value comprises detecting a correspondence between the heart rate variability and the respiration rate variability.

**Patentansprüche**

1. Vorrichtung zum Bestimmen eines medizinischen Zustands eines menschlichen Subjekts, wobei die Vorrichtung umfasst:

eine Steuereinheit; und
ein Mittel, um Pulswellendaten bereitzustellen, die für einen Herzschlag des menschlichen Subjekts repräsentativ sind; wobei
die Steuereinheit ausgestaltet ist, um die Schritte durchzuführen:

Empfangen der Pulswellendaten;
Auswählen eines Anteils der Pulswellendaten, der eine Vielzahl von Herzperioden angibt;
für den Anteils der Pulswellendaten, der eine Vielzahl von Herzperioden angibt:

- Bestimmen einer Blutdruckvariabilität basierend auf den Pulswellendaten des Anteils der Pulswellendaten, der eine Vielzahl von Herzperioden angibt;
- Bestimmen einer Atemfrequenzvariabilität basierend auf den Pulswellendaten des Anteils der Pulswellendaten, der eine Vielzahl von Herzperioden angibt; und
- Bestimmen einer Herzfrequenzvariabilität basierend auf den Pulswellendaten des Anteils der Pulswellendaten, der eine Vielzahl von Herzperioden angibt; und

Bestimmen mindestens eines Korrelationswerts basierend auf mindestens einer von der Blutdruckvariabilität, der Atemfrequenzvariabilität, der Herzfrequenzvariabilität und einem jeweiligen Referenzwert; und Bestimmen eines medizinischen Zustands des Subjekts basierend auf dem mindestens einen Korrelationswert; wobei
das Subjekt eine Körpergröße, ein Alter und ein Geschlecht aufweist, und
der Schritt des Bestimmens der Blutdruckvariabilität Bestimmen einer Vielzahl von Blutdruckwerten umfasst,
der Schritt des Bestimmens einer Vielzahl von Blutdruckwerten für jeden jeweiligen Blutdruckwert der Vielzahl der Blutdruckwerte Folgendes umfasst, wobei jeder jeweilige Blutdruckwert einer jeweiligen Herzperiode von der Vielzahl der Herzperioden zugeordnet ist:

- Bestimmen einer systolischen Komponente der jeweiligen Herzperiode;
- Annähern an die systolische Komponente mit einer ersten Gauß-Funktion und einer zweiten Gauß-Funktion; und
- Bestimmen einer Zeitdifferenz (WWT) zwischen der ersten und der zweiten Gauß-Funktion; und

Bestimmen eines jeweiligen Blutdruckwerts (BP) von der Vielzahl der Blutdruckwerte des Subjekts basierend auf der Zeitdifferenz (WWT), der Körpergröße und/oder dem Alter; und wobei der Schritt des Bestimmens einer Vielzahl von Blutdruckwerten des Weiteren für jeden Blutdruckwert von der Vielzahl der Blutdruckwerte umfasst:

- Bestimmen eines vorläufigen Steifheitsindex (SI$_p$) basierend auf der Körpergröße und der Zeitdifferenz (WWT);
- Bestimmen eines bereinigten Steifheitsindex (SI$_a$) basierend auf dem vorläufigen Steifheitsindex (SI$_p$) und dem Alter; und
- Bestimmen des Blutdruckwerts (BP) basierend auf dem bereinigten Steifheitsindex (SI$_a$) und einem Regressionsmodell.

2. Vorrichtung nach dem vorhergehenden Anspruch, wobei die Pulswellendaten, die eine Vielzahl von Herzperioden angeben, sich auf eine Vielzahl von Herzperioden beziehen, die direkt aufeinander folgen.

3. Vorrichtung nach einem der vorhergehenden Ansprüche, wobei der Schritt des Bestimmens der Atemfrequenzvariabilität basierend auf den Pulswellendaten des Anteils der Pulswellendaten, der eine Vielzahl von Herzperioden angibt, umfasst:

Bestimmen einer Vielzahl von Maxima basierend auf den Pulswellendaten, wobei die Vielzahl der Maxima die maximale Amplitude einer jeweiligen Vielzahl von Herzperioden bezeichnet;
Bestimmen eines Atemsignals, das die Atemfrequenz angibt, basierend auf der Vielzahl von Maxima, wobei gegebenenfalls Bestimmen des Atemsignals basierend auf einer Spline-Interpolation der Vielzahl von Maxima eingeschlossen ist; und
Bestimmen der Atemfrequenzvariabilität basierend auf einer Zeitdifferenz zwischen jedem Maximum des Atemsignals.

4. Vorrichtung nach einem der vorhergehenden Ansprüche, wobei der Schritt des Bestimmens der Herzfrequenzvariabilität basierend auf den Pulswellendaten des Anteils der Pulswellendaten, der eine Vielzahl von Herzperioden angibt, umfasst:

Bestimmen einer Vielzahl von Referenzpunkten basierend auf den Pulswellendaten, wobei die Vielzahl der Referenzpunkte einer jeweiligen Komponente der Vielzahl von Herzperioden entspricht, wobei die jeweilige Komponente gegebenenfalls eine von einer Maximalamplitude der Herzperiode, einer steigenden Flanke der Herzfrequenzamplitude ist;
Bestimmen der Herzfrequenzvariabilität basierend auf einer Zeitdifferenz zwischen jedem Referenzpunkt von der Vielzahl der Referenzpunkte.

5. Vorrichtung nach einem der vorhergehenden Ansprüche, wobei

- der Anteil der Pulswellendaten eine Vielzahl von Herzperioden angibt, und wobei der Schritt des Bestimmens der Zeitdifferenz (WWT) des Weiteren umfasst:

Bestimmen der Zeitdifferenz (WWT) für die Vielzahl aufeinander folgender Herzperioden als Mittelwert basierend auf den jeweiligen Zeitdifferenzen, die für die Vielzahl der Herzperioden bestimmt werden; wobei der Mittelwert gegebenenfalls der Medianwert der bestimmten jeweiligen Zeitdifferenzen ist; und/oder wobei

- die erste und die zweite Gauß-Funktion eine jeweilige Maximalamplitude aufweisen, wobei die Maximalamplitude der ersten Gauß-Funktion größer als oder gleich der Maximalamplitude der zweiten Gauß-Funktion ist; und/oder wobei
- die erste und die zweite Gauß-Funktion jeweilige erste und zweite Standardabweichungen ($\sigma_1$, $\sigma_2$) aufweisen, wobei die erste und die zweite Standardabweichung ($\sigma_1$, $\sigma_2$) die gleichen sind.

6. Vorrichtung nach einem der vorhergehenden Ansprüche, wobei der Schritt des Annäherns an die systolische Komponente umfasst: Anpassen der ersten und der zweiten Gauß-Funktion an die systolische Komponente unter Verwendung von:

$$F(a,b,c,d,f) = \sum_{i=1}^{N} \left( S_i - \left( a \cdot e^{-\frac{1}{2}\left(\frac{t-b}{c}\right)^2} + d \cdot e^{-\frac{1}{2}\left(\frac{t-f}{c}\right)^2} \right) \right)^2 \stackrel{!}{=} min$$

wobei *a, b, c* und *d* unter Verwendung von nichtlinearer Optimierung oder Kurvenanpassung bestimmt werden.

7. Vorrichtung nach einem der vorhergehenden Ansprüche, wobei das Regressionsmodell eine Regressionsfunktion

$$f(SI_a, g) = BP_{sys},$$

umfasst, in der $SI_a$ der bereinigte Steifheitsindex ($SI_a$) ist, g das Geschlecht des Subjekts ist, und $BP_{sys}$ der Blutdruck ist; und wobei Bestimmen des Blutdruckwertes Bestimmen des Blutdruckwertes basierend auf der Regressionsfunktion umfasst, wobei die Regressionsfunktion eine lineare Funktion des Typs

$$f(x) = ax + b$$

umfasst, wobei a im Bereich von 1 bis 20 mmHg/ (m/s) liegt und *b* im Bereich von 0 bis 80 mmHg liegt, wobei a bevorzugter im Bereich von 5 bis 15 mmHg/ (m/s) liegt, und *b* im Bereich von 20 bis 60 mmHg liegt.

8. Vorrichtung nach einem der vorhergehenden Ansprüche, wobei Bestimmen des bereinigten Steifheitsindex ($SI_a$) auf einer Bereinigungsfunktion

$$f\ (SI_p)\ =\ SI_a$$

basiert, wobei $SI_p$ der vorläufige Steifheitsindex ist, und $SI_a$ der bereinigte Steifheitsindex ($SI_a$) ist, wobei die Bereinigungsfunktion gegebenenfalls eine lineare Funktion des Typs

$$f(x)\ =\ cx\ +\ d$$

ist, wobei c und d Bereinigungsfaktoren sind, die basierend auf einer Vielzahl von Wertepaaren bestimmt werden, die einen Alterswert und einen dazugehörigen Steifheitsindexwert umfassen; wobei gegebenenfalls:

$$c = \frac{SI - \mu}{Bereich(Alter)}$$

wobei $\mu$ = 0,109 * *Alter* + 3,699 und *Bereich (Alter) = 0,1663 * Alter* + 4,3858 - $\mu$, wobei *Alter* das Alter des Subjekts ist, und
d = 0.

**9.** Vorrichtung nach einem der vorhergehenden Ansprüche, wobei das Bestimmen der systolischen Komponente umfasst:

Bestimmen eines jeweiligen globalen Maximums der jeweiligen Herzperiode;
Bestimmen der Ableitung zweiter Ordnung der jeweiligen Herzperiode;
Bestimmen eines Maximalwerts der Ableitung zweiter Ordnung, der sich in mindestens einer vorbestimmten Zeitdifferenz zu dem globalen Maximum befindet; und
Definieren der systolischen Komponente als Anteil der Herzperiode zwischen dem Start der Herzperiode und dem Maximalwert; wobei gegebenenfalls die vorbestimmte Zeitdifferenz zu dem globalen Maximum 350 ms oder weniger beträgt, wobei des Weiteren gegebenenfalls die vorbestimmte Zeitdifferenz zu dem globalen Maximum 250 ms oder weniger beträgt.

**10.** Vorrichtung nach einem der vorhergehenden Ansprüche, wobei Bestimmen des vorläufigen Steifheitsindex ($SI_p$) auf einer Funktion

$$SI_p = \frac{h}{WWT}$$

basiert, wobei *h* die Größe des Subjekts ist, *WWT* die Zeitdifferenz ist, und $SI_p$ der vorläufige Steifheitsindex ($SI_p$) ist.

**11.** Vorrichtung nach einem der vorhergehenden Ansprüche, wobei der Schritt des Bestimmens von mindestens einem Korrelationswert auf der Herzfrequenzvariabilität basiert; wobei der Schritt des Bestimmens von mindestens einem Korrelationswert des Weiteren umfasst:

Generieren einer Häufigkeitsverteilung, welche die Verteilung der Vielzahl von Herzfrequenzvariabilitätswerten in der Zeitdomäne angibt, basierend auf einer Vielzahl von Herzfrequenzvariabilitätswerten;
Bestimmen einer Vielzahl von erwarteten Werten;
Bestimmen eines Entropiewerts, der eine Vielzahl von erwarteten Werten angibt, wobei der Entropiewert den medizinischen Zustand des Subjekts angibt.

**12.** Vorrichtung nach dem vorhergehenden Anspruch, wobei die Häufigkeitsverteilung, welche die Verteilung der Vielzahl von Herzfrequenzvariabilitätswerten angibt, ein Histogramm umfasst, wobei das Histogramm gegebenenfalls eine Bin-Größe von 8 ms aufweist.

**13.** Vorrichtung nach einem der vorhergehenden Ansprüche, wobei der Anteil der Pulswellendaten, der eine Vielzahl von Herzperioden angibt, eine Periode zwischen 2 Minuten und 5 Minuten abdeckt; und der Schritt des Bestimmens der Variabilitäten des Blutdrucks, der Atemfrequenz und der Herzfrequenzvariabilität auf im Wesentlichen allen Herzschlägen basiert, die in den Pulswellendaten enthalten sind.

**14.** Vorrichtung nach einem der vorhergehenden Ansprüche, wobei der Schritt des Bestimmens von mindestens einem Korrelationswert auf der Herzfrequenzvariabilität und der Atemfrequenzvariabilität basiert, und wobei der Schritt des Bestimmens von mindestens einem Korrelationswert Detektieren einer Entsprechung zwischen der Herzfrequenzvariabilität und der Atemfrequenzvariabilität umfasst.

**Revendications**

**1.** Appareil permettant de déterminer un état médical d'un sujet humain, l'appareil comprenant :

une unité de commande ; et
un moyen permettant de fournir des données d'onde d'impulsion représentatives d'un battement cardiaque du sujet humain ;
l'unité de commande étant configurée pour réaliser les étapes de :

réception des données d'onde d'impulsion ;
sélection d'une partie des données d'onde d'impulsion indicative d'une pluralité de périodes cardiaques ;
pour la partie des données d'onde d'impulsion indicative d'une pluralité de périodes cardiaques :

- détermination d'une variabilité de pression sanguine sur la base des données d'onde d'impulsion de la partie des données d'onde d'impulsion indicative d'une pluralité de périodes cardiaques ;
- détermination d'une variabilité de fréquence respiratoire sur la base des données d'onde d'impulsion de la partie des données d'onde d'impulsion indicative d'une pluralité de périodes cardiaques ; et
- détermination d'une variabilité de fréquence cardiaque sur la base des données d'onde d'impulsion de la partie des données d'onde d'impulsion indicative d'une pluralité de périodes cardiaques ; et

détermination d'au moins une valeur de corrélation sur la base d'au moins une variable parmi : la variabilité de pression sanguine, la variabilité de fréquence respiratoire, la variabilité de fréquence cardiaque, et une valeur de référence respective ; et
détermination d'un état médical du sujet sur la base de l'au moins une valeur de corrélation ;
le sujet ayant une hauteur de corps, un âge et un sexe, et
l'étape de détermination de variabilité de pression sanguine comprenant la détermination d'une pluralité de valeurs de pression sanguine, l'étape de détermination d'une pluralité de valeurs de pression sanguine comprenant, pour chaque valeur de pression sanguine respective de la pluralité de valeurs de pression sanguine, chaque valeur de pression sanguine respective étant associée à une période cardiaque respective de la pluralité de périodes cardiaques :

- détermination d'une composante systolique de la période cardiaque respective ;
- approximation de la composante systolique avec une première fonction gaussienne et une seconde fonction gaussienne ; et
- détermination d'une différence de temps (WWT) entre les première et seconde fonctions gaussiennes ; et

détermination d'une valeur de pression sanguine (BP) respective parmi la pluralité de valeurs de pression sanguine du sujet sur la base de la différence de temps (WWT), de la hauteur de corps et/ou de l'âge ; et l'étape de détermination d'une pluralité de valeurs de pression sanguine comprenant en outre, pour chaque valeur de pression sanguine de la pluralité de valeurs de pression sanguine :

- la détermination d'un indice de rigidité préliminaire ($SI_p$) sur la base de la hauteur de corps et de la différence de temps (WWT) ;
- la détermination d'un indice de rigidité ajusté ($SI_a$) sur la base de l'indice de rigidité préliminaire ($SI_p$) et de l'âge ; et
- la détermination de la valeur de pression sanguine (BP) sur la base de l'indice de rigidité ajusté ($SI_a$) et d'un modèle de régression.

**2.** Appareil selon la revendication précédente, les données d'onde d'impulsion indicatives d'une pluralité de périodes cardiaques se rapportant à une pluralité de périodes cardiaques se succédant directement les unes aux autres.

**3.** Appareil selon l'une quelconque des revendications précédentes, l'étape de détermination de la variabilité de fréquence respiratoire sur la base des données d'onde d'impulsion de la partie des données d'onde d'impulsion indicatives d'une pluralité de périodes cardiaques comprenant :

la détermination d'une pluralité de maxima sur la base des données d'onde d'impulsion, la pluralité de maxima indiquant l'amplitude maximale d'une pluralité respective de périodes cardiaques ;
la détermination d'un signal respiratoire indicatif de la fréquence respiratoire sur la base de la pluralité de maxima, comprenant éventuellement la détermination du signal respiratoire sur la base d'une interpolation de fonction spline de la pluralité de maxima ; et
la détermination de la variabilité de fréquence respiratoire sur la base d'une différence de temps entre chaque maximum du signal respiratoire.

**4.** Appareil selon l'une quelconque des revendications précédentes, l'étape de détermination de la variabilité de fréquence cardiaque sur la base des données d'onde d'impulsion de la partie des données d'onde d'impulsion indicatives d'une pluralité de périodes cardiaques comprenant :

la détermination d'une pluralité de points de référence sur la base des données d'onde d'impulsion, la pluralité de points de référence correspondant à une composante respective de la pluralité de périodes cardiaques, la composante respective étant éventuellement l'une d'une amplitude maximale de la période cardiaque, d'un front montant de l'amplitude de fréquence cardiaque ;
la détermination de la variabilité de fréquence cardiaque sur la base d'une différence de temps entre chaque point de référence de la pluralité de points de référence.

**5.** Appareil selon l'une quelconque des revendications précédentes,

- la partie des données d'onde d'impulsion étant indicative d'une pluralité de périodes cardiaques, et l'étape de détermination de la différence de temps (WWT) comprenant en outre :
la détermination de la différence de temps (WWT) pour la pluralité de périodes cardiaques successives en tant que valeur moyenne sur la base des différences de temps respectives déterminées pour la pluralité de périodes cardiaques ; éventuellement la valeur moyenne étant la valeur médiane des différences de temps respectives déterminées ; et/ou
- les première et seconde fonctions gaussiennes ayant une amplitude maximale respective, l'amplitude maximale de la première fonction gaussienne étant supérieure ou égale à l'amplitude maximale de la seconde fonction gaussienne ; et/ou
- les première et seconde fonctions gaussiennes ayant des premier et second écarts-types respectifs ($\sigma_1$, $\sigma_2$), les premier et second écarts-types ($\sigma_1$, $\sigma_2$) étant égaux entre eux.

**6.** Appareil selon l'une quelconque des revendications précédentes, l'étape d'approximation de la composante systolique comprenant :

l'ajustement des première et seconde fonctions gaussiennes à la composante systolique à l'aide de

$$F(a,b,c,d,f) = \sum_{i=1}^{N} \left( S_i - \left( a \cdot e^{-\frac{1}{2}\left(\frac{t-b}{c}\right)^2} + d \cdot e^{-\frac{1}{2}\left(\frac{t-f}{c}\right)^2} \right) \right)^2 \overset{!}{=} min$$

*a, b, c* et *d* étant déterminés à l'aide d'une optimisation non linéaire ou d'un ajustement de courbe.

**7.** Appareil selon l'une quelconque des revendications précédentes, le modèle de régression comprenant une fonction de régression

$$f(SI_a, g) = BP_{sys},$$

où $SI_a$ est l'indice de rigidité ajusté ($SI_a$), g est le sexe du sujet, et $BP_{sys}$ est la pression sanguine ; et la détermination de la valeur de pression sanguine comprenant la détermination de la valeur de pression sanguine sur la base de la fonction de régression, la fonction de régression comprenant une fonction linéaire du type

$$f(x) = ax + b,$$

a variant de 1 à 20 mmHg/ (m/s) et *b* variant de 0 à 80 mmHg, plus préférablement a variant de 5 à 15 mmHg/(m/s) et *b* variant de 20 à 60 mmHg.

8.  Appareil selon l'une quelconque des revendications précédentes, la détermination de l'indice de rigidité ajusté ($SI_a$) étant basée sur une fonction d'ajustement $f(SI_p) = SI_a$, où $SI_p$ est l'indice de rigidité préliminaire et $SI_a$ est l'indice de rigidité ajusté ($SI_a$), la fonction d'ajustement étant éventuellement une fonction linéaire du type

$$f(x) = cx + d,$$

où *c* et *d* sont des facteurs d'ajustement déterminés sur la base d'une pluralité de paires de valeurs comprenant une valeur d'âge et une valeur d'indice de rigidité associée ; éventuellement :

$$c = \frac{SI - \mu}{amplitude(\hat{a}ge)}$$

avec $\mu$ = 0,109 * *âge* + 3,699 et *amplitude(âge)* = 0,1663 * *âge + 4,3858* - $\mu$, *âge* étant l'âge du sujet, et *d* = 0.

9.  Appareil selon l'une quelconque des revendications précédentes,
    la détermination de la composante systolique comprenant :

    la détermination d'un maximum global respectif de la période cardiaque respective ;
    la détermination de la dérivée de second ordre de la période cardiaque respective ;
    la détermination d'une valeur maximale de la dérivée de second ordre située au moins à une différence de temps prédéterminée par rapport au maximum global ; et
    la définition de la composante systolique en tant que partie de la période cardiaque entre le début de la période cardiaque et la valeur maximale ; éventuellement, la différence de temps prédéterminée par rapport au maximum global étant de 350 ms ou moins, en outre, éventuellement, la différence de temps prédéterminée par rapport au maximum global étant de 250 ms ou moins.

10. Appareil selon l'une quelconque des revendications précédentes, la détermination de l'indice de rigidité préliminaire ($SI_p$) étant basée sur une fonction

$$SI_p = \frac{h}{WWT},$$

où *h* est la hauteur de sujet, WWT est la différence de temps, et $SI_p$ est l'indice de rigidité préliminaire ($SI_p$) .

11. Appareil selon l'une quelconque des revendications précédentes, l'étape de détermination d'au moins une valeur de corrélation étant basée sur la variabilité de fréquence cardiaque ; l'étape de détermination d'au moins une valeur de corrélation comprenant en outre :

    la génération, sur la base d'une pluralité de valeurs de variabilité de fréquence cardiaque, d'une distribution de fréquence indicative de la distribution de la pluralité de valeurs de variabilité de fréquence cardiaque dans le domaine temporel ;
    la détermination d'une pluralité de valeurs attendues ;
    la détermination d'une valeur d'entropie indicative d'une pluralité de valeurs attendues, la valeur d'entropie étant indicative de l'état médical du sujet.

12. Appareil selon la revendication précédente, la distribution de fréquence indicative de la distribution de la pluralité de valeurs de variabilité de fréquence cardiaque comprenant un histogramme, éventuellement l'histogramme ayant une taille d'intervalle de 8 ms.

13. Appareil selon l'une quelconque des revendications précédentes, la partie des données d'onde d'impulsion indicatives d'une pluralité de périodes cardiaques couvrant une période comprise entre 2 minutes et 5 minutes ; et l'étape de détermination des variabilités de la pression sanguine, de la fréquence respiratoire et de la variabilité de fréquence cardiaque, étant basée sur sensiblement tous les battements cardiaques compris dans les données d'onde d'impulsion.

14. Appareil selon l'une quelconque des revendications précédentes, l'étape de détermination d'au moins une valeur de corrélation étant basée sur la variabilité de fréquence cardiaque et la variabilité de fréquence respiratoire, et l'étape de détermination d'au moins une valeur de corrélation comprenant la détection d'une correspondance entre la variabilité de fréquence cardiaque et la variabilité de fréquence respiratoire.

FIG.1

FIG.2

300 — ( START )

Recording
pulse wave data — 302

Determining suitable
heart rate periods — 304

Decomposition of each heart period
into systolic and diastolic components — 306

Approximation of the systolic component
with at least two Gaussian functions by means of
fitting against the original pulse wave — 308

Determining the time difference
between the fitted Gaussian functions — 310

Determining the SI from the height of
the subject and the time difference — 312

Compensating the determined SI value
based on the age of the subject by means of
known linear dependencies — 314

Estimation of the effective blood pressure
as a function of the compensated SI by means of
gender-specific regression models — 316

318 — ( END )

FIG.3A

EP 3 117 766 B1

207

RESPIRATION

201

MEASURED
PULSE WAVE

EP 3 117 766 B1

BLOOD PRESSURE
& VARIATION

HEART RATE
& VARIATION

208

206

FIG.3B

FIG.3C

# FIG.4

400 — ( START )

↓

| Record video signal indicative of acral blood flow using video sensor and light source of mobile device | —402 |

↓

| Choose region of interest of at least 50x50 pixels | —404 |

↓

| Eliminate 1st Bit of green value of pixel and calculate sum of values for each frame to obtain associated sample | —406 |

↓

| Obtain time stamp for region of interest based on respective video frame | —408 |

↓

| Obtain pulse wave signal based on sample | —410 |

↓

| Re-sample pulse wave signal based on samples and time stamps using a cubic spline interpolation in order to obtain re-sampled pulse wave signal | —412 |

↓

| Filter re-sampled pulse wave signal to eliminate noise and to compensate for drift | —414 |

↓

| Obtain original pulse wave signal for processing | —416 |

↓

418 — ( END )

FIG.5

FIG.5A

EP 3 117 766 B1

## FIG.6

## FIG.7

## FIG.8

FIG.9A

FIG.9B

## REFERENCES CITED IN THE DESCRIPTION

### Patent documents cited in the description

- US 2010228102 A
- US 2013079606 A1
- WO 2014031082 A
- US 8398556 B

### Non-patent literature cited in the description

- **A. SEECK ; W. RADEMACHER ; C. FISCHER ; J. HAUEISEN ; R. SURBER ; A. VOSS.** *Prediction of atrial fibrillation recurrence after cardioversion-Interaction analysis of cardiac autonomic regulation*
- **W. POPPE et al.** *Eignen sich die Hüllungskurven von Arterienpulswellen für eine Fernbeurteilung psychotischer Krankheitsverläufe?*